(19) Europäisches Patentamt
European Patent Office
Office européen des brevets

(11) Veröffentlichungsnummer: **0 019 132**
**A1**

# (12) EUROPÄISCHE PATENTANMELDUNG

(21) Anmeldenummer: **80102291.4**

(22) Anmeldetag: **28.04.80**

(51) Int. Cl.³: **C 07 C 49/457**
C 07 C 49/467

(30) Priorität: **08.05.79 DE 2918468**

(43) Veröffentlichungstag der Anmeldung:
**26.11.80 Patentblatt 80/24**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Hübner, Armin, Dr.**
**Breslauer Strasse 31**
**D-4150 Krefeld(DE)**

(72) Erfinder: **Heine, Hans-Georg, Dr.**
**Am Heckerhof 14**
**D-4150 Krefeld(DE)**

(72) Erfinder: **Hartmann, Willy, Dr.**
**Hohenzollernstrasse 25**
**D-4150 Krefeld(DE)**

(54) **Verfahren zur Herstellung von alpha-Halogencyclobutanonen.**

(57) Die vorliegende Erfindung betrifft ein Verfahren zur Herstellung von α-Halogencyclobutanonen der allgemeinen Formel (I)

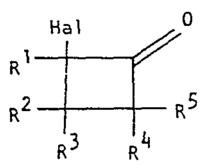

(I)

worin $R^1$ bis $R^5$ die in der Beschreibung angegebene Bedeutung besitzen, das dadurch gekennzeichnet ist, daß ein Cyclobutanon der allgemeinen Formel (II)

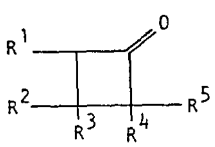

(II)

in einem aprotischen Verdünnungsmittel, mit einem substituierten, gegebenenfalls in situ erzeugten, Ammoniumperhalogenid bei Temperaturen von -20°C bis +60°C, gegebenenfalls in Anwesenheit eines Katalysators umgesetzt wird.

EP 0 019 132 A1

Croydon Printing Company Ltd.

BAYER AKTIENGESELLSCHAFT 5090 Leverkusen, Bayerwerk
Zentralbereich
Patente, Marken und Lizenzen
Rt-by
Typ IVa

# Verfahren zur Herstellung von α-Halogencyclobutanonen

Gegenstand der Erfindung ist ein Verfahren zur Herstellung von α-Halogencyclobutanonen durch Halogenierung von Cyclobutanonen mit substituierten Ammoniumperhalogeniden.

Zur Herstellung von α-Halogencyclobutanonen sind verschiedene Verfahren bekannt.

So bilden sich α-Halogencyclobutanone durch Cycloaddition von α-Halogenketenen, z.B. α-Chlorketenen, an Olefine. Hierbei ist es zur Erzielung guter Ausbeuten notwendig, das α-Chlorketen in situ zu erzeugen (DE-OS 2 539 048 und 2 813 337). Ein anderes Verfahren bedient sich der bekannten α-Halogenierung cyclischer Ketone. Danach werden beispielsweise Cyclobutanon und seine methylierten Derivate durch Bromierung in Chloroform in Gegenwart von Bromwasserstoff in die entsprechenden α-Bromcyclobutanone übergeführt (Bull. Soc. Chim. Fr. 1964, 1957; 773). Nachteil dieser Umsetzung ist die Bildung von α,α-Dibromcyclobutanonen, deren Anteil im Reaktionsprodukt beträchtlich sein kann. Da solche α,α-dihalogenierten Cyclobutanone mit Alkalilaugen bzw. -alkoholaten unter Ringöffnung reagieren,

sind sie für die Herstellung von Cyclopropancarbonsäuren ungeeignet.

Nachteilig bei der Umsetzung von vinylsubstituierten Cyclobutanonen mit Halogenen kann ferner sein, daß das Halogen bevorzugt mit der Vinylgruppe reagiert und die erwünschte α-Halogenierung zurückgedrängt oder sogar unterdrückt wird. Ein solcher Fall tritt bei einem Cyclobutanon mit einer durch drei Halogenatome substituierten Vinylgruppe naturgemäß mit einer erheblich geringeren Wahrscheinlichkeit ein, kann aber bei der Umsetzung eines (Monochlor)-vinyl-substituierten Cyclobutanons zur dominierenden Reaktion werden.

Die Verwendung anderer, üblicher Halogenierungsmittel, wie beispielsweise N-Bromsuccinimid oder Sulfurylchlorid, führt bei vinylsubstituierten Cyclobutanonen zu einer unerwünschten Allylhalogenierung.

Die vorliegende Erfindung betrifft ein Verfahren zur Herstellung von α-Halogencyclobutanonen der allgemeinen Formel (I)

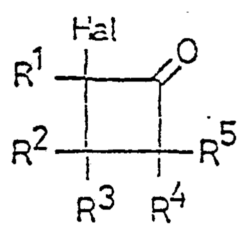

(I)

worin

$R^1$-$R^5$ gleich oder verschieden sind und Wasserstoff, jeweils gegebenenfalls substituiertes Alkyl, Cycloalkyl, Aryl, Aralkyl und Alkenyl bedeuten oder ferner

$R^2$ und $R^3$ und/oder $R^4$ und $R^5$ mit den Atomen, mit denen sie verbunden sind, einen gegebenenfalls substituierten carbocyclischen Ring mit 3-6 C-Atomen bilden und

Hal für Chlor oder Brom steht,

das dadurch gekennzeichnet ist, daß ein Cyclobutanon der allgemeinen Formel (II)

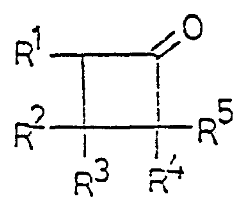

(II)

worin

$R^1$-$R^5$ und Hal die voranstehend genannte Bedeutung haben,

in einem aprotischen Verdünnungsmittel, mit einem substituierten, gegebenenfalls in situ erzeugten, Ammoniumperhalogenid bei Temperaturen von -20° bis +60°, gegebenenfalls in Gegenwart eines Katalysators umgesetzt wird.

Die α-Halogencyclobutanone werden nach dem erfindungsgemäßen Verfahren in guter Ausbeute und hoher Reinheit erhalten. Die Halogenierung der Doppelbindung, Allylhalogenierung sowie Bildung des α,α-Dihalogenids werden zurückgedrängt bzw. sogar unterdrückt. Dies war überraschend.

Die für die Halogenierung nach dem erfindungsgemäßen Verfahren geeigneten Cyclobutanone der Formel (II) sind bekannt oder werden nach bekannten Verfahren erhalten. (s. D. Seebach in Houben-Weyl, Methoden der Organischen Chemie, Vol. 4/4, G. Thieme, Stuttgart 1971; J. Am. Chem. Soc. 94, 2870 (1972); Angew. Chem. 86, 272 (1974); Angew. Chem. 87, 552 (1975); DOS 2 654 062; DOS 2 813 337).

Bevorzugt werden Verbindungen der Formel (II) im erfindungsgemäßen Verfahren eingesetzt, in denen die Reste $R^1$-$R^5$ für gegebenenfalls substituiertes geradkettiges oder verzweigtes Alkyl mit bis zu 12, bevorzugt bis zu 8, insbesondere 1 und 2 C-Atomen steht, für gegebenenfalls substituiertes Alkenyl mit 2-12, bevorzugt 2-6 C-Atomen, insbesondere der Vinyl- und Allylrest ferner für gegebenenfalls substituiertes Aryl mit bis zu 14 C-Atomen, insbesondere der Phenylrest, sowie für Aralkyl, insbesondere Benzyl- und Phenylethyl, sowie für gegebenenfalls substituiertes Styryl.

Als Substituenten der gegebenenfalls substituierten Reste $R^1$ bis $R^5$ seien beispielsweise genannt: Cyan, Halogen, insbesondere Chlor oder Brom, Alkoxyreste, Alkylsulfonyl, Phenyl, das gegebenenfalls durch Halogen substituiert ist.

Besonders bevorzugte Cyclobutanone der Formel (II) sind:
2,2,3,3-Tetramethylcyclobutanon
2,2,3-Trimethylcyclobutanon
2,2-Dimethylcyclobutanon
2,2,3-Trimethyl-3-phenylcyclobutanon
2,2,3-Trimethyl-3-(2-chlorphenyl)-cyclobutanon
2,2-Diphenylcyclobutanon
2,2-Dimethyl-3-vinyl-cyclobutanon
2,2-Diethylcyclobutanon
2,2-Diethyl-3-vinyl-cyclobutanon
Spiro[3,5]nonanon-2
Spiro[3,4]octanon-2
Spiro[3,3]heptanon-2
Spirohexanon-4
4-(ß,ß-Dichlorvinyl)-spiro[3,4]octanon-2
4-(ß,ß-Dichlorvinyl)-spiro[3,3]heptanon-2
2-Ethyl-2-methyl-cyclobutanon
2-(ß,ß-Dichlorvinyl)-3,3-dimethyl-cyclobutanon
2,2-Dimethyl-3-(α-methyl-ß,ß-dichlorvinyl)-cyclobutanon
2,2-Diethyl-3-(α,ß-dichlorvinyl)-cyclobutanon

2,2-Dimethyl-3-(α,β,β-trifluorvinyl)-cyclobutanon
2,2-Diethyl-3-(α,β,β-trichlorvinyl)-cyclobutanon
2,2-Dimethyl-3-(α,β-dichlorvinyl)-cyclobutanon
2,2-Dimethyl-3-(β,β-dibromvinyl)-cyclobutanon
2,2-Dimethyl-3-(α-fluor-β,β-dichlorvinyl)-cyclobutanon
2,2-Dimethyl-3-(β-chlorvinyl)-cyclobutanon
2,2,3-Trimethyl-3-(α,β,β-trifluorvinyl)-cyclobutanon
2,2-Dimethyl-3-(β,β-difluorvinyl)-cyclobutanon
2-Ethyl-2-methyl-3-(β,β-dichlorvinyl)-cyclobutanon
2-Ethyl-2-methyl-3-(α,β,β-trichlorvinyl)-cyclobutanon
2-Ethyl-2,3-dimethyl-3-(α,β,β-trichlorvinyl)-cyclobutanon
2,2-Diethyl-3-(β,β-dibromvinyl)-cyclobutanon
2-Ethyl-2-methyl-3-(β,β-dibromvinyl)-cyclobutanon
2-Ethyl-2-methyl-3-(α-fluor-β,β-dichlorvinyl)-cyclobutanon
2,2-Dimethyl-3-(α-ethyl-β,β-dichlorvinyl)-cyclobutanon
2-Ethyl-2,3-dimethyl-3-(β,β-dichlorvinyl)-cyclobutanon
4-(β,β-Dichlorvinyl)-spiro/3,5/-nonanon-2
2,2-Dimethyl-3-(α,β-dibromvinyl)-cyclobutanon
4-(α,β,β-Trichlorvinyl)-spiro/3,5/-nonanon-2
4-(β,β-Dibromvinyl)-spiro/3,5/-nonanon-2
2,2-Dimethyl-3-(β-brom-β-chlorvinyl)-cyclobutanon
2,2-Dimethyl-4-ethyl-3-(β,β-dichlorvinyl)-cyclobutanon
2,2,4-Trimethyl-3-(α,β-dibromvinyl)-cyclobutanon
2,2-Dimethyl-4-n-butyl-3-(β,β-dichlorvinyl)-cyclobutanon
2-Methyl-3-(α,β,β-trichlorvinyl)-cyclobutanon

Die als Halogenierungsmittel verwendeten Ammoniumperhalogenide sind bekannt. Besonders bevorzugt sind

Pyridiniumperbromid (Pyridinhydrobromidperbromid); Phenyl-trimethylammoniumperbromid; Benzyl-trimethylammoniumperbromid; Chinoliniumperbromid; ein Perbromid aus Pyrrolidon, Bromwasserstoff und Brom (J. Org. Chem. 28, 573 (1963); ein mit einem Polymer verknüpftes Benzyl-trimethylammoniumperbromid (Synthesis 1979, 64) Pyridiniumchlorid/Chlor; Pyridiniumchlorid/Brom.

Als bevorzugte Verdünnungsmittel für die Durchführung der erfindungsgemäßen Reaktion seien folgende inerte aprotische organische Lösungsmittel genannt: Ester von Mono- und Dicarbonsäuren, wie z.B. Essigsäureethylester, Essigsäuremethylester, Propionsäuremethylester, Bernsteinsäurediethylester, Ameisensäure-n-butylester, Ameisensäuremethylester, Ameisensäureethylester, Dimethylcarbonat, Bernsteinsäuredimethylester oder Ether, wie z.B. Diethylether, Di-iso-propylether, Dioxan, Tetrahydrofuran, Dimethylether, Methylbutylether, oder Amide, wie z.B. Dimethylacetamid.

Als Katalysatoren die zur Durchführung der erfindungsgemäßen Reaktion gegebenenfalls verwendet werden können seien bevorzugt Halogenwasserstoffsäuren wie Bromwasserstoff, Chlorwasserstoff; Tetrabutylammoniumbromid, Pyridiniumbromid, Pyridiniumchlorid genannt.

Die Reaktionstemperatur der Halogenierung ist in einem relativ weiten Bereich wählbar. Als präparativ nützlich erweist sich ein Temperaturbereich von -20° bis +60°, vorzugsweise 20 bis 50°.

Die erfindungsgemäße Reaktion wird bevorzugt so durchgeführt, daß das Cyclobutanon in einem Verdünnungsmittel gegebenenfalls zusammen mit einem Katalysator vorgelegt wird und das Halogenierungsmittel portionsweise in die Lösung eingetragen wird, wobei die Geschwindigkeit der Zugabe sich nach dem Umsatz des Halogenierungsmittels richtet, d.h. erst wenn zuvor eingetragenes Halogenierungsmittel umgesetzt ist, erfolgt die Zugabe einer weiteren Menge Halogenierungsmittel. Eine andere Methode besteht darin, daß man die Reaktanden (Cyclobutanonderivat und Halogenierungsmittel, Lösungsmittel und gegebenenfalls Katalysator) zusammengibt und bei 20 bis 50° reagieren läßt. Eine weitere Variante besteht darin, daß ein Teil des sich während der Reaktion bildenden Halogenwasserstoffs mit Stickstoff aus der Reaktionslösung ausgetrieben oder durch Umsetzung mit einer basischen Verbindung, wie z.B. Calciumcarbonat oder Soda, entfernt wird. Schließlich kann die Halogenierung auch so durchgeführt werden, daß das Cyclobutanon und ein substituiertes Ammoniumhalogenid in einem geeigneten Verdünnungsmittel vorgelegt und das Perhalogenid in situ durch Zugabe des Halogens erzeugt wird.

Die Aufarbeitung der Reaktionslösung kann so erfolgen, daß der Halogenwasserstoff mit Stickstoff oder Luft ausgetrieben wird und die Reaktionslösung gegebenenfalls nach Entfernen des überschüssigen Halogenierungsmittels sowie des ausgeschiedenen Hydrohalogenids der tertiären Stickstoffbase direkt in die Umsetzung mit einer wäßrigen Alkalibase zu der entsprechenden Cyclopropancarbonsäureester dieses niederen Alkohols eingesetzt wird. Das rohe α-Halogenketon läßt sich durch Waschen der Reaktionslösung mit Wasser, gegebenenfalls unter Zusatz eines mit Wasser nicht mischbaren Lösungsmittels, halogenwasserstofffrei erhalten und durch Kristallisieren oder Destillieren in reiner Form isolieren.

Das bei der Umsetzung nach dem erfindungsgemäßen Verfahren gebildete,oftmals in der Lösung nicht oder schwerlösliche Hydrohalogenid des tert. Amins läßt sich einfach abfiltrieren und nach Halogenierung (L.F. Fieser und M. Fieser, Reagents for Organic Synthesis, <u>1967</u>, 967) wieder in das Perhalogenid (z.B. Pyridiniumperbromid) umwandeln. Ebenso lassen sich die tetrasubstituierten Ammoniumhalogenide nach Abtrennen durch Halogenierung, insbesondere Bromierung, in die tetrasubstituierten Ammoniumperhalogenide (insbesondere Bromide) überführen.

Die nach dem erfindungsgemäßen Verfahren leicht zugänglichen α-Halogencyclobutanone, insbesondere die α-Bromcyclobutanone der allgemeinen Formel (I) sind

Zwischenprodukte zur Herstellung insektizider Wirkstoffe. So läßt sich z.B. das 4-Brom-2,2-dimethyl-3-(ß,ß-dichlorvinyl)-cyclobutanon mit dem Natriumsalz des m-Phenoxybenzylalkohols in Toluol glatt zum m-Phenoxybenzylester der 2,2-Dimethyl-3-(ß,ß-dichlorvinyl)-cyclopropancarbonsäure (Permethrin) umsetzen.

Beispiel 1

In einem 250 ml Dreihalskolben, versehen mit Innenthermometer, Gaseinleitungsrohr, Rührer und Rückflußkühler, werden 9,65 g (0,05 mol) 2,2-Dimethyl-3-(ß,ß-dichlorvinyl)-cyclobutanon in 150 ml Essigsäureethylester gelöst. Unter Durchleiten von Stickstoff werden bei 20° portionsweise insgesamt 16,0 g (0,05 mol) Pyridiniumperbromid (Pyridinhydrobromidperbromid) zugegeben. Nach 12 h Rühren filtriert man das abgeschiedene, schwerlösliche Pyridinhydrobromid ab, dampft die Lösung im Vakuum ein und destilliert den Rückstand. 12,0 g hellgelbes Öl vom Sdp.$_{0,25}$ 80-82°. Die gaschromatographische Analyse liefert folgende Zusammensetzung: 86,2 % 4-Brom-2,2-dimethyl-3-(ß,ß-dichlorvinyl)-cyclobutanon, 10,7 % 2,2-Dimethyl-3-(ß,ß-dichlorvinyl)-cyclobutanon, 3,1 % 4,4 Dibrom-2,2-dimethyl-3-(ß,ß-dichlorvinyl)-cyclobutanon. Durch fraktionierende Destillation an einer Ringspaltkolonne lassen sich die drei Komponenten trennen. 4-Brom-2,2-dimethyl-3-(ß,ß-dichlorvinyl)-cyclobutanon Sdp.$_{0,6}$ 95° (Isomerengemisch: trans:cis = 81:19).

Zum Vergleich wurde die aus (Bull. Soc. Chim. Fr. 1964, 1976) bekannte Halogenierung von 2,2-Dimethylcyclobutanon nach dem dort angegebenen Verfahren durchgeführt:

Die Lösung von 61,0 g (0,62 mol) 2,2-Dimethylcyclobutanon in 250 ml Tetrachlorkohlenstoff, der ca. 5 % Bromwasserstoff enthält, wird bei 20° tropfenweise so mit insgesamt

100,0 g (0,62 mol) Brom in 250 ml Tetrachlorkohlenstoff versetzt, daß die Lösung vor erneuter Bromzugabe jeweils entfärbt ist. Nach Zugabe der gesamten Menge Brom wird 30 Minuten nachgerührt und gelöster Bromwasserstoff durch Einleiten von Stickstoff ausgetrieben. Aus dem NMR-Spektrum der Lösung läßt sich entnehmen, daß im Verhältnis von ca. 2:1:2.2 2,2-Dimethylcyclobutanon, sein α-mono- und sein α,α-dibromiertes Derivat vorliegen. Man wäscht die Lösung 3 mal mit je 50 ml gesättigter wäßriger Kochsalzlösung und destilliert fraktionierend an einer Ringspaltkolonne:

25,7 g 2,2-Dimethylcyclobutanon (Sdp.$_{100}$ 51-54$^{o}$)
23,3 g 4-Brom-2,2-dimethylcyclobutanon (Sdp.$_{14}$ 74-75$^{o}$)
47,3 g 4,4-Dibrom-2,2-dimethylcyclobutanon (Sdp.$_{14}$ 90-91$^{o}$, Schmp. 41,5 - 42$^{o}$)

<u>Beispiel 2</u>

Zu der Lösung von 9,65 g (0,05 mol) 2,2-Dimethyl-3-(ß,ß-dichlorvinyl)-cyclobutanon in 150 ml Essigsäureethylester werden 8,8 g (0,05 mol) Pyridinhydrobromid gegeben. Innerhalb von 2 h tropft man bei 20$^{o}$ 8,0 g (0,05 mol) Brom in die Suspension und rührt anschließend 12 h nach. Aufarbeitung, wie unter Beispiel 1 beschrieben, liefert 11,8 g eines gelbbraunen Öls, dessen Zusammensetzung gaschromatographisch bestimmt wurde:

85-86 % 4-Brom-2,2-dimethyl-3-(ß,ß-dichlorvinyl)-cyclobutanon
10-11 % 2,2-Dimethyl-3-(ß,ß-dichlorvinyl)-cyclobutanon
3-4 % 4,4-Dibrom-2,2-dimethyl-3-(ß,ß-dichlorvinyl)-cyclobutanon.

Beispiel 3

9,8 g (0,1 mol) 2,2-Dimethylcyclobutanon werden in 300 ml Essigsäureethylester bei 20° mit 32,0 g (0,1 mol) Pyridiniumperbromid, wie in Beispiel 1 beschrieben, umgesetzt. Durch Destillation werden 12,9 g farbloses Öl vom $Sdp._{9}$ 68-72° erhalten, das laut gaschromatographischer Analyse zu 90 % aus 4-Brom-2,2-dimethylcyclobutanon sowie zu 10 % aus 4,4-Dibrom-2,2-dimethylcyclobutanon besteht. Das Destillat enthält kein 2,2-Dimethylcyclobutanon.

Beispiel 4

7,9 g (0,05 mol) 2,2-Dimethyl-3-(ß-chlorvinyl)-cyclobutanon werden in 180 ml Essigsäureethylester bei 20° mit 16,0 g (0,05 mol) Pyridiniumperbromid, wie in Beispiel 1 beschrieben, umgesetzt. Man erhält durch fraktionierende Destillation 9,5 g gelbliches Öl vom $Sdp._{0,05}$ 65-70°.
Gaschromatographische Analyse:

73 % 4-Brom-2,2-dimethyl-trans-3-(ß-chlorvinyl)-cyclobutanon

13 % 4-Brom-2,2-dimethyl-cis-3-(ß-chlorvinyl)-cyclobutanon

9 % 2,2-Dimethyl-3-(ß-chlorvinyl)-cyclobutanon

5 % höher bromierte Produkte.

Beispiel 5

11,8 g (0,05 mol) 2,2-Dimethyl-3-(2'-(4',4'-dichlor-2'-methyl-but-3'-enyl)-cyclobutanon werden in 150 ml Essigsäureethylester bei 20° mit 16,0 g (0,05 mol) Pyridiniumperbromid, wie in Beispiel 1 beschrieben, umgesetzt. Es werden 13,0 g farbloses Öl vom $Sdp._{0,02}$ 84° erhalten. Gaschromatographische Analyse:

88,6 % 4-Brom-2,2-dimethyl-3-(2'(4',4'-dichlor-2'-methyl-but-3'-enyl))-cyclobutanon

7,5 % 2,2-Dimethyl-3-(2'-(4',4'-dichlor-2'-methyl-but-3'-enyl))-cyclobutanon

2,9 % höher bromierte Produkte.

2,2-Dimethyl-3-(2'-(4',4'-dichlor-2'-methyl-but-3'-enyl)).-cyclobutanon wird in Anlehnung an das aus DE-OS 2 654 062 bekannte Verfahren durch Cycloaddition von Dimethylketenimoniumchlorid an 1,1-Dichlor-3,3-dimethyl-penta-1,4-dien in Gegenwart von wasserfreiem Zinkchlorid und folgender Hydrolyse hergestellt ($Sdp._{0,05}$ 74°).

Beispiel 6

34,23 g (0,126 mol) 4-Brom-2,2-dimethyl-3-(ß,ß-dichlorvinyl)-cyclobutanon werden bei 10° unter Zusatz von 5 ml Methylenchlorid in 200 ml 2n NaOH 24 h gerührt. Extrahieren der wäßrig alkalischen Lösung mit Ether liefert 0,25 g Neutralprodukt. Man säuert die wäßrige Lösung unter Kühlen mit konzentrierter Salzsäure an und extrahiert sie anschließend 3 mal mit je 100 ml Ether. Die Etherphase wird 1 mal mit 50 ml gesättigter Kochsalzlösung gewaschen, über wasserfreiem Natriumsulfat getrocknet und eingedampft. Man erhält 25,67 g kristalline 2,2-Dimethyl-3-(ß,ß-dichlorvinyl)-cyclopropancarbonsäure als Isomerengemisch (trans:cis = 82:18).

Beispiel 7

Zu der Lösung von 2,3 g (0,1 mol) Natrium in 80 ml abs. Ethanol gibt man unter Stickstoff 20,0 g (0,1 mol) m-Phenoxybenzylalkohol und destilliert bei 40°/20 Torr Ethanol ab. Den Rückstand versetzt man 2 mal mit je 80 ml abs. Toluol und dampft bei 50°/20 Torr zur Trockne ein. Nach Zugabe von 150 ml abs. Toluol tropft man bei 15-20° in die Suspension die Lösung von 30,0 g (0,11 mol) 4-Brom-2,2-dimethyl-3-(ß,ß-dichlorvinyl)-cyclobutanon in 120 ml abs. Toluol und rührt 6 h nach. Anschließend erhitzt man 30 Minuten zum Rückfluß und

gibt darauf den Reaktionsansatz auf Eis/10 % Salzsäure. Die organische Phase wird abgetrennt, bis zur Neutralreaktion mit gesättigter Natriumbicarbonatlösung und Wasser gewaschen, getrocknet und eingedampft. Den öligen Rückstand (37,1 g) chromatographiert man an 600 g Kieselgel und erhält neben geringen Mengen Permethrinsäureethylester 31,2 g Permethrinsäure-m-phenoxybenzylester.

<u>Beispiel 8</u>

Gemäß Beispiel 1 werden 11,06 g (0,05 mol) 2,2-Diethyl-3-(ß,ß-dichlorvinyl)-cyclobutanon in 150 ml Ameisensäureethylester mit 16,0 g (0,05 mol) Pyridiniumperbromid bei 20° umgesetzt. Aufarbeitung liefert 16,2 g gelbliches Öl vom Siedebereich 90-95°/0,3-0,4 Torr. Die gaschromatographische Analyse des Destillats ergibt einen Gehalt von 92 % 2,2-Diethyl-3-(ß,ß-dichlorvinyl)-4-bromcyclobutanon-Isomerengemisch.

<u>Beispiel 9</u>

15,0 g (0,08 mol) 2,2,3-Trimethyl-3-phenylcyclobutanon werden, wie in Beispiel 1 beschrieben, in 200 ml Essigsäure-n-butylester mit 25,6 g (0,08 mol) Pyridiniumperbromid bei 20° umgesetzt. Aufarbeitung liefert 20,72 g hellbraunes Öl, dessen NMR-spektroskopische Analyse

($CDCl_3$ als Lösungsmittel) hinweist, daß 2,2,3-Trimethyl-3-phenyl-4-brom-cyclobutanon (Isomerengemisch) als Hauptprodukt vorliegt.
Ber. % Br 79,92
Gef. 79,0

Beispiel 10

5,18 g (0,025 Mol) 2,2,3-Trimethyl-3-(ß,ß-dichlorvinyl)-cyclobutanon werden in 70 ml Essigsäureethylester bei 20° mit 8,0 g (0,025 mol) Pyridiniumperbromid umgesetzt. Aufarbeitung, wie in Beispiel 1 beschrieben, liefert 8,7 g hellbraunes Öl vom $Sdp._{0,9}$ 110-115°. Der Anteil an 2,2,3-Trimethyl-3-(ß,ß-dichlorvinyl)-4-brom-cyclobutanon (Isomerengemisch) im Destillat beträgt 84 %.

Beispiel 11

11,66 g (0,05 mol) 4-(ß,ß-Dichlorvinyl)-spiro[3,5]nonanon-2 in 170 ml Essigsäuremethylester werden bei 20° mit 16,0 g (0,05 mol) Pyridiniumperbromid, wie in Beispiel 1 beschrieben, umgesetzt. Aufarbeitung liefert 17,8 g rohes Bromketon mit einem Gehalt von 91 % 4-(ß,ß-Dichlorvinyl)-3-brom-spiro[3,5]nonanon-2 (Isomerengemisch).

Beispiel 12

Gemäß Beispiel 1 werden 9,65 g (0,05 mol) 2,2-Dimethyl-3-(ß,ß-dichlorvinyl)-cyclobutanon in 180 ml Essigsäuremethylester bei 20° mit 18,8 g (0,05 mol) Phenyl-trimethylammoniumperbromid umgesetzt. Man erhält 12,6 g hellgelbes Öl von $Sdp._{0,3}$ 80°, das zu über 95 % aus 4-Brom-2,2-dimethyl-3-(ß,ß-dichlorvinyl)-cyclobutanon besteht.

Beispiel 13

Gemäß Beispiel 1 werden 9,65 g (0,05 mol) 2,2-Dimethyl-3-(ß,ß-dichlorvinyl)-cyclobutanon in 150 ml Essigsäureethylester bei 20° mit 21,3 g (0,043 mol) des Perbromids, hergestellt aus 3 mol Pyrrolidon, 1 mol Brom und 1 mol Bromwasserstoff, umgesetzt. Man erhält 11,6 g hellgelbes Öl, das folgende Komponenten enthält:

9 % 2,2-Dimethyl-3-(ß,ß-dichlorvinyl)-cyclobutanon
62 % 4-Brom-2,2-dimethyl-3-(ß,ß-dichlorvinyl)-cyclobutanon
29 % 4,4-Dibrom-2,2-dimethyl-3-(ß,ß-dichlorvinyl)-cyclobutanon.

Beispiel 14

Gemäß Beispiel 1 werden 9,65 g (0,05 mol) 2,2-Dimethyl-3-(ß,ß-dichlorvinyl)-cyclobutanon in 180 ml Diethylcarbonat mit 16,0 g (0,05 mol) Pyridiniumperbromid umgesetzt. Man erhält 11,8 g farbloses Öl vom $Sdp._{0,2-0,3}$

70-78°, das aus 85 % 4-Brom-2,2-dimethyl-3-(ß,ß-dichlorvinyl)-cyclobutanon und 15 % 4,4-Dibrom-2,2-dimethyl-3-(ß,ß-dichlorvinyl)-cyclobutanon enthält.

Wird anstelle des Diethylcarbonats Ameisensäureethylester (180 ml) als Verdünnungsmittel verwendet und die Umsetzung unter sonst gleichen Bedingungen durchgeführt, so erhält man 11,77 g schwach gelb gefärbtes Öl vom $Sdp._{0,2}$ 75°, das nur das gesuchte 4-Brom-2,2-dimethyl-3-(ß,ß-dichlorvinyl)-cyclobutanon enthält.

Patentansprüche

1. Verfahren zur Herstellung von α-Halogencyclobutanonen der allgemeinen Formel (I)

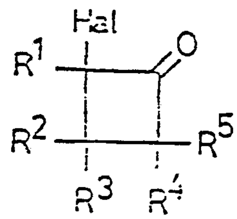

(I)

in welcher

$R^1$-$R^5$ gleich oder verschieden sind und Wasserstoff, jeweils gegebenenfalls substituiertes Alkyl, Cycloalkyl, Aryl, Aralkyl und Alkenyl bedeuten oder ferner

$R^2$ und $R^3$ und/oder $R^4$ und $R^5$ mit den Atomen, mit denen sie verbunden sind, einen gegebenenfalls substituierten carbocyclischen Ring mit 3-6 C-Atomen bilden und

Hal für Chlor oder Brom steht,

dadurch gekennzeichnet, daß ein Cyclobutanon der allgemeinen Formel (II)

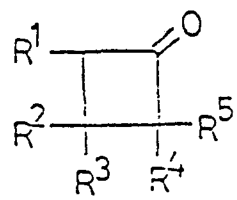

(II)

in welcher

$R^1$-$R^5$ und Hal die voranstehend genannte Bedeutung haben,

in einem aprotischen Verdünnungsmittel, mit einem substituierten, gegebenenfalls in situ erzeugten Ammoniumperhalogenid bei Temperaturen von $-20^o$ bis $+60^o$, gegebenenfalls in Gegenwart eines Katalysators umgesetzt wird.

2. Verfahren nach Anspruch 1, dadurch gekennzeichnet, daß als Cyclobutanon ein 2,2-Dimethyl-3-(ß,ß-dihalovinyl)-cyclobutanon, als Halogenierungsmittel Pyridiniumperbromid und als Verdünnungsmittel Ameisen- oder Essigsäureethylester verwendet wird.

3. Verfahren nach Anspruch 1, dadurch gekennzeichnet, daß als Cyclobutanon ein 2,2-Dimethyl-3-(ß-halovinyl)-cyclobutanon, als Halogenierungsmittel

Pyridiniumperbromid und als Verdünnungsmittel Ameisen- oder Essigsäureethylester verwendet wird.

Europäisches Patentamt

# EUROPÄISCHER RECHERCHENBERICHT

Nummer der Anmeldung

EP 80 10 2291.4

## EINSCHLÄGIGE DOKUMENTE

| Kategorie | Kennzeichnung des Dokuments mit Angabe soweit erforderlich, der maßgeblichen Teile | betrifft Anspruch | KLASSIFIKATION DER ANMELDUNG (Int.Cl.3) |
|---|---|---|---|
| A,D | DE - A1 - 2 654 062 (BAYER)<br>* Seite 12, Position 3/1 *<br>-- | 1 | C 07 C 49/457<br>C 07 C 49/467 |
| A | Chemical Abstracts Band 70 Nr. 13, 31. März 1969<br>Columbus, Ohio, USA<br>D.V.C. AWANG et al. "Pyrrolidinone hydrotribromide: brominating agent with selectivity for ketones"<br>Seite 273, Spalte 1, Abstract Nr. 56890c<br>& Can. J. Chem. Band 47, Nr. 4, 1969, Seiten 706 bis 709 (Eng)<br>-- | 2,3 | RECHERCHIERTE SACHGEBIETE (Int. Cl.3)<br>C 07 C 49/457<br>C 07 C 49/467 |
| A | THE JOURNAL OF ORGANIC CHEMISTRY, Band 42, Nr. 6, 18. März 1977<br>Columbus, Ohio, USA<br>W.K. ANDERSON et al. "Synthesis of 6,9-Bisnormethyl-8-Methoxy-12,13-Epoxy-6,8,10-Trichothecatriene"<br>Seiten 1045 bis 1050<br>* Seiten 1048 und 1050 *<br>-- | 1 | |
| | BULLETIN OF THE CHEMICAL SOCIETY OF JAPAN, Band 52, Nr. 2, Februar 1979<br>Tokio<br>N.J. REDDY et al. "Synthesis and Reduction of 3-Bromoflavanones"<br>Seiten 647 bis 648<br>---- | 2,3 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 21-08-1980 | KNAACK |